# EUROPEAN PATENT APPLICATION

(11) **EP 3 981 249 A1**
(43) Date of publication of application: **13.04.2022**
(21) Application number: 20200917.1
(22) Date of filing: 08.10.2020
(51) Int. Cl.: A01N 25/10, A01N 33/12, A01N 39/00, A01N 57/34, A61L 2/00, A01N 59/00, C08F 220/60, C09D 133/26, A01P 1/00

(54) **SANITISING FORMULATION**

(71) Applicant: Lumendo AG, 1020 Renens (CH)
(72) Inventor: Johnson, Aaron, 8400 Winterthur (CH); Philippe, Léa, 8052 Zürich (CH); Bispinghoff, Mark, 5080 Zürich (CH); Schmocker, Andreas, 1018 Lausanne (CH)
(74) Representative: GatesIP

(57) **Abstract**

The present invention provides a polymerizable sanitizing formulation for forming a polymerized anti-microbial agent retaining material on a contact surface. The polymerizable sanitizing formulation comprising: a) a polymerizable water-soluble material; b) at least one initiator for radical polymerization of the polymerizable material to form a polymerized anti-microbial agent retaining material; c) at least one anti-microbial agent for retention within the polymerized anti-microbial agent retaining material; and d) at least one solvent.

## Description

The present invention relates to a sanitising formulation, in particular a polymerizable sanitising formulation, for use in sanitising an area or surface, such as for example hands of a user, and to a method for producing the sanitising formulation.

### BACKGROUND OF INVENTION

There has been an increasing awareness among the general population for the need to clean hands and surfaces regularly in order to prevent the spread of infections or diseases through a community.

Conventional methods for cleaning hands and surfaces involve washing hands with soap, the use of alcohol-based hand sanitizers, cleaning contact surfaces (such as hard surfaces in kitchens and bathrooms) with disinfectants, bleach or surface cleaners, and protecting hands with gloves or surfaces with protective covers.

Even with regular hand washing, the skin can become contaminated within minutes upon contact with a contaminated surface, leading to self-contamination, infection and spread of the disease. It is not always possible to have access to hand washing facilities during the course of a day and there are concerns that public restrooms increase the spread of a disease or infection. Gloves merely provide a protection against hand-to-face infections. Therefore, there is an increasing demand for hand sanitizers which can be carried by people in order to use frequently during the course of the day to help reduce the risk of contamination. There is also an increased need for hand sanitizers at high population footfall areas such as for example hospitals, schools, workplaces, shopping centres and transport hubs.

Conventional hand sanitizers have a short lifespan once applied to the hands of a user. Conventional hand sanitizers are alcohol-based and although alcohol is an effective antimicrobial, once applied to the skin of a user, alcohol evaporates quickly leaving the skin unprotected. Conventional hand sanitizer might also contain non-volatile disinfectants can be washed away by water or sweat from the pores of the skin of a user. As a result, the loss of the alcohol or disinfectant can lead to recontamination which leads to a greater risk of spread of infection or disease.

There is therefore a need for a hand sanitizer with an improved lifespan compared to conventional hand sanitizers. There is a need for a non-alcohol-based hand sanitizer.

Additionally, the aforementioned material could potentially show promise when applied to other surfaces. Thus, imbuing these surfaces with anti-microbial properties.

### SUMMARY OF INVENTION

According to a first aspect there is provided a polymerizable sanitizing formulation for forming a polymerized anti-microbial agent retaining material retaining at least one anti-microbial agent therein, the polymerizable sanitizing formulation comprising:
a) a polymerizable water-soluble material;
b) at least one initiator for radical polymerization of the polymerizable material to form a polymerized anti-microbial agent retaining material;
c) at least one anti-microbial agent for retention within the polymerized anti-microbial agent retaining material; and
d) at least one solvent.

The term "polymerizable water-soluble material" is used herein to refer to mater which is soluble in water and which has at least one polymerizable group capable of undergoing polymerization (preferably radical polymerization) to form a polymerized material.

The term "polymerized anti-microbial agent retaining material" is used herein to refer to a polymerized material which retains at least one anti-microbial agent within the material. The polymerized anti-microbial agent retaining material may be for example a polymerized anti-microbial agent retaining hydrogel polymer.

The term "hydrogel polymer" is used herein to refer to a polymer gel formed from a network of cross-linked hydrophilic polymer chains, the cross-links consisting of physical crosslinks (e.g. hydrogen bonding, hydrophobic interactions, and chain entanglements) and chemical crosslinks. The polymer gel is capable of retaining a large volume of water while maintaining structural integrity.

The term "anti-microbial agent" is used herein to refer to an agent that can destroy or slow or prevent growth and development of microbes. Examples of anti-microbial agents include: antibacterial agents, antiviral agents, antifungal agents and antiparasitic agents.

The term "initiator" is used herein to refer to a compound that is configured to react with a polymerizable material to form an intermediate compound capable of linking successively with a one or more other polymerizable materials to form a polymerized material.

In one embodiment, the polymerized anti-microbial agent retaining material is formed on a contact surface. The term "contact surface" is used herein to a surface configured to be in contact with the polymerized anti-microbial agent retaining material, such as for example, but not to be limited to, hard surfaces (such as kitchen surfaces and/or appliances, bathroom surfaces and/or appliances, floors, woodwork, metalwork, domestic appliances, industrial appliances), soft surfaces (such as for example textiles and/or laundry), skin and/or hair etc.

The polymerizable water-soluble material preferably comprises one or more materials selected from:
PEG and/or PPG and/or copolymers thereof, comprising one or more radically polymerizable end groups;
one or more natural polymeric material(s) and/or polymers derived from the Extra Cellular Matrix (ECM), such as for example one or more of: gelatin, elastin, collagen, agar/agarose, chitosan, fibrin, proteoglycans;
one or more polyamino-acid and/or derivatives thereof, preferably polylysin or gelatin methyl cellulose, carbomethyl cellulose;
one or more polysaccharides and their derivatives, preferably glycosaminoglycanes such as hyaluronic acid, chondroitinsulfate, dermatansulfate, heparansulfate, heparine, keratansulfate or alginate;
one or more nucleotides, polylipids, fatty acids, poly lactic acid, lactic acid, cationic polyallylammonium chloride; and/or
any derivative and/or fragment and/or combination thereof.

Preferably, the polymerizable water-soluble material comprises one or more of: PEG, PPG, or copolymer moieties thereof comprising one or more radically polymerizable end groups.

In one embodiment, the polymerizable sanitizing formulation comprises:
a) 3 to 50 % in weight of a polymerizable water-soluble material;
b) 0.001 - 5 % in weight of at least one initiator for radical polymerization of the polymerizable material to form a polymerized anti-microbial agent retaining material;
c) 0.001 - 10 % in weight of at least one anti-microbial agent for retention within the polymerized anti-microbial agent retaining material; and
d) 10 - 90 % in weight of at least one solvent.

In one embodiment, the polymerizable sanitizing formulation further comprises:
e) at least one coinitiator to assist the initiator in radical polymerization of the polymerizable water-soluble material.

In one embodiment, the coinitiator(s) is present within the formulation in an amount of 0.01 - 5% in weight compared to the total weight of the formulation.

In one embodiment, the polymerizable sanitizing formulation is a photopolymerizable sanitizing formulation in which the polymerizable water-soluble material is a photopolymerizable water-soluble material. Furthermore, in one embodiment, the at least one initiator is at least one photo-initiator. The photopolymerizable sanitizing formulation is polymerizable on exposure to a light source to form a polymerized anti-microbial agent retaining material.

The term "photo-initiator" is used herein to refer to a molecule which creates reactive species (for example free radicals, cations or anions) when exposed to light radiation (for example visible or UV light).

In one embodiment, the polymerizable sanitizing formulation, for example the photopolymerizable sanitizing formulation, further comprises:
f) at least one antioxidant.

Preferably, the formulation comprises 0.01 - 5 % in weight of at least one antioxidant compared to the total weight of the formulation.

In one embodiment, the polymerizable sanitizing formulation, for example the photopolymerizable sanitizing formulation, further comprises:
g) a non-polymerizable water-soluble material comprising PEG, PPG, or copolymer moieties thereof.

The term "non-polymerizable water-soluble material" is used herein to refer to a water-soluble material which does not contain a polymerizable group, and is not capable of being polymerized (i.e. is not capable of forming covalent bonds with another material to form a polymer chain).

The non-polymerizable water-soluble material preferably comprises one or more materials selected from:
PEG and/or PPG and/or copolymers thereof;
one or more natural polymeric material(s) and/or polymers derived from the Extra Cellular Matrix (ECM), such as for example one or more of: gelatin, elastin, collagen, agar/agarose, chitosan, fibrin, proteoglycans;
one or more polyamino-acid and/or derivatives thereof, preferably polylysin or gelatin methyl cellulose, carbomethyl cellulose;
one or more polysaccharides and their derivatives, preferably glycosaminoglycanes such as hyaluronic acid, chondroitinsulfate, dermatansulfate, heparansulfate, heparine, keratansulfate or alginate;
one or more nucleotides, polylipids, fatty acids, poly lactic acid, lactic acid, cationic polyallylammonium chloride; and/or
any derivative and/or fragment and/or combination thereof.

Preferably, the formulation comprises 3 - 50 % in weight of a non-polymerizable water-soluble material comprising PEG, PPG, or copolymer moieties thereof compared to the total weight of the formulation.

In one embodiment, the polymerizable sanitizing formulation (preferably the photopolymerizable sanitizing formulation) comprises:
a) 3 - 50 % in weight of a polymerizable water-soluble material (preferably the polymerizable water-soluble material comprises PEG, PPG, or copolymer moieties thereof, comprising one or more radically polymerizable end groups);
b) 0.001 - 5 % in weight of at least one initiator (preferably at least one photoinitiator) for radical polymerization of the polymerizable material to form a polymerized anti-microbial agent retaining material;
c) 0.001 -10 % in weight of at least one anti-microbial agent for retention within the polymerized anti-microbial agent retaining material;
d) 10 - 90 % in weight of at least one solvent;
e) 0.01 - 5 % in weight of at least one coinitiator to assist the initiator (for example photoinitiator) in radical polymerization of the polymerizable material;
f) 0.01 - 5 % in weight of at least one antioxidant; and
g) 3 - 50 % in weight of a non-polymerizable water-soluble material comprising PEG, PPG, or copolymer moieties thereof;

In one embodiment, the polymerizable sanitizing formulation (for example photopolymerizable sanitizing formulation) comprises 5 - 20 % in weight of a polymerizable water-soluble material, preferably 5 -15 % in weight of a polymerizable water-soluble material compared to the total weight of the formulation.

In one embodiment, the polymerizable sanitizing formulation (for example photopolymerizable sanitizing formulation) comprises 5 - 20 % in weight of a polymerizable water-soluble material, comprising PEG, PPG, or copolymer moieties thereof, comprising one or more radically polymerizable end groups, preferably 5 - 15 % in weight of a polymerizable water-soluble material comprising PEG, PPG, or copolymer moieties thereof, comprising one or more radically polymerizable end groups, compared to the total weight of the formulation.

The number-average molecular weight of the polymerizable water-soluble material comprising PEG, PPG, or copolymer moieties thereof, comprising one or more radically polymerizable end groups, are preferably within the range of from 2000 to 100000 g/mol, preferably within the range of from 6000 to 30000 g/mol, preferably within the range of from 1000 to 15000 g/mol, for example approximately 1000 g/mol.

The polymerizable water-soluble material comprising PEG, PPG, or copolymer moieties thereof, comprising one or more radically polymerizable end groups, comprises one or more radically polymerizable end group that can be polymerized by radical polymerization. Preferably, the polymerizable water-soluble material comprising PEG, PPG, or copolymer moieties thereof, comprising one or more radically polymerizable end groups, comprises one or more radically polymerizable end groups consisting of: acrylate; methacrylate; acrylamide; methacrylamide; but-3-en-2-one; inverse methacrylate of formula (I): 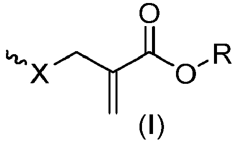 where R is an alkyl, X = O, NH; vinylsulfone; vinylurea; vinylcarbonate; vinylcarbamate; vinylthioester; vinylthiourea.

In one embodiment, the polymerizable sanitizing formulation (for example the photopolymerizable sanitizing formulation) comprises 5 - 20 % in weight of a non-polymerizable water-soluble material comprising PEG, PPG, or copolymer moieties thereof, preferably 5 -15 % in weight of a non-polymerizable water-soluble material comprising PEG, PPG, or copolymer moieties thereof compared to the total weight of the formulation.

The number-average molecular weight of the non-polymerizable water-soluble material comprising PEG, PPG, or copolymer moieties thereof are preferably within the range of from 2000 to 100000 g/mol, preferably within the range of from 6000 to 30000 g/mol, preferably within the range of from 1000 to 20000 g/mol, for example approximately 1000 g/mol.

If the polymerizable water-soluble material and/or the non-polymerizable water-soluble material comprises a copolymer, then the composition of the PEG and PPG parts of the copolymers within the polymerizable and/or non-polymerizable material may be adjusted. The composition can comprise of (but are not limited to) one or more copolymer groups of poloxamer, inverse poloxamer, pluronic, inverse pluronic, poloxamine, inverse poloxamine, tetronic, and inverse tetronic. For example, the composition of the PEG-PPG copolymers within the polymerizable and/or non-polymerizable material may be adjusted to increase the ratio of PEG to PPG or to decrease the ratio of PEG to PPG as required provided that the copolymers are water-soluble. In one embodiment, the polymerizable water-soluble material and/or the non-polymerizable water-soluble material comprises a copolymer, and the composition of the PEG part within the PEG/PPG part copolymers is within the range of 50 - 90 % in weight of PEG.

The generation of free radicals for polymerization can be triggered thermally by decomposition of a thermally unstable compound (thermal initiator) or photochemically (photoinitiator). It is well understood to a person skilled in the art, that the thermal activation barrier of thermal initiators can be lowered by addition of other compounds, resulting in so-called "redox" or "two-component" initiators or initiation systems. The term "initiator" is used herein to refer to include, but not to be limited to, thermal initiators, photoinitiators and "redox" or "two-component" initiators or initiation systems.

In one embodiment, the polymerizable sanitizing formulation (for example photopolymerizable sanitizing formulation) comprises 0.01 - 2% in weight of at least one initiator (for example persulfate derivatives, azo derivatives, peroxide derivatives, peracid derivatives, peracetate derivatives, and hydroperoxide derivatives optionally in combination with a reducing agent resulting in so-called "redox" or "two-component" initiators or initiation systems, quinones, α-hydroxy ketones, acylgermanium derivatives, bis(acyl)phosphine oxide derivatives, mono(acyl)phosphine oxide derivatives, xanthene derivatives, xanthone derivatives, thioxanthone derivatives, fluorescein derivatives, rhodamine derivatives, triarylmethane derivatives, phenoxazine derivatives, phenazine derivatives, azo derivatives, phenothiazine derivatives, anthraquinone derivatives, cyanine derivatives, curcumin derivatives, and coumarin derivatives, or any combination thereof) compared to the total weight of the formulation for radical polymerization of the polymerizable material to form a polymerized anti-microbial agent retaining material.

In one embodiment, the at least one initiator is at least one photo-initiator capable of polymerising the polymerizable material on exposure to light, preferably ambient light. The photoinitiator may be selected from one or more of: common UV, violet, blue or other visible light active photoinitiators or dyes. Preferably, the one or more photo-initiator is selected from the group comprising: quinones, α-hydroxy ketones, acylgermanium derivatives, bis(acyl)phosphine oxide derivatives, mono(acyl)phosphine oxide derivatives, xanthene derivatives, xanthone derivatives, thioxanthone derivatives, fluorescein derivatives, rhodamine derivatives, triarylmethane derivatives, phenoxazine derivatives, phenazine derivatives, azo derivatives, phenothiazine derivatives, anthraquinone derivatives, cyanine derivatives, curcumin derivatives, and coumarin derivatives, or any combination thereof.

In one embodiment, the polymerizable sanitizing formulation (for example photopolymerizable sanitizing formulation) comprises 0.01 - 5 % in weight, preferably 0.1 - 1 % in weight compared to the total weight of the formulation, of at least one anti-microbial agent for retention within the polymerized anti-microbial agent retaining material.

In one embodiment, the polymerizable sanitizing formulation (for example photopolymerizable sanitizing formulation) comprises 0.1 - 2 % in weight of at least one coinitiator compared to the total weight of the formulation to assist the initiator in radical polymerization of the polymerizable material.

The coinitiator may be selected from one or more Norrish Type 2 coinitiators or accelerators for radical polymerization. The coinitiator is preferably selected from one or more of: primary, secondary, or tertiary amines or anilines with the proviso that an α-hydrogen must be present, or diaryl iodonium salts, or any combination thereof.

In one embodiment, the polymerizable sanitizing formulation (for example photopolymerizable sanitizing formulation) comprises 0.1 - 2 % in weight of a least one antioxidant compared to the total weight of the formulation.

The antioxidant may be selected from one or more phenols, nitroxides, phosphines, sulfides, silanes, polyanilines, or any combination thereof. The antioxidant is preferably selected from one or more of: unsubstituted or substituted phenols (preferably methyl, ethyl or tert-butyl substituted phenols), monoarylphosphines, diarylphosphines, triarylphosphines, or arylsulfides, or any combination thereof. Preferably, the antioxidant is selected from one or more of: monoarylphosphines, diarylphosphines, or triarylphosphines, or any combination thereof.

In one embodiment, the polymerizable sanitizing formulation (for example photopolymerizable sanitizing formulation) comprises 50 - 80 % in weight of at least one solvent compared to the total weight of the formulation.

The suitable solvent is preferably a non-reactive solvent which is a solvent that does not (co)polymerize into the polymer or interfere with the polymerization reaction. The solvent is preferably selected from one or more of: water, acetone, DMSO, alcohols, or any combination thereof.

The resultant polymerized anti-microbial agent retaining material is preferably a polymerized anti-microbial agent retaining hydrogel polymer, for example a polymerized anti-microbial agent retaining cross-linked hydrogel polymer.

The anti-microbial agent(s) may comprise any suitable anti-microbial agent(s) or combinations thereof. Examples of suitable anti-microbial agents include those that do not exhibit strongly oxidizing, acidic, or basic properties as their primary mechanism of disinfection.

Preferably, the anti-microbial agent(s) comprises any amphiphilic ionic anti-microbial agent.

Preferably, the anti-microbial agent(s) is selected from one or more of: povidone iodine, 2-phenylphenol, chlorocresol, octenidine dihydrochloride, mecetronium ethylsulfate, didecyldimethylammonium chloride, bisbiguanide derivatives, polyhexamethylene guanidine derivatives, benzethonium chloride, quaternary ammonium compounds (QACs), for example benzalkonium species (Formula IV): 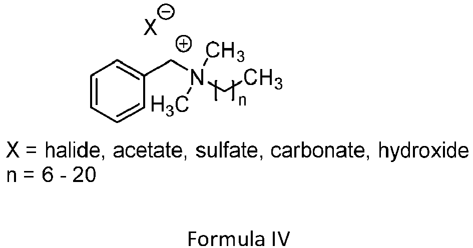 , or any combination thereof.

The anti-microbial agent(s) is present within the formulation at an amount of 0.001 - 30 % in weight, preferably present within the formulation at an amount of 0.001 - 10 % in weight, preferably present within the formulation at an amount of 0.01 - 3 % in weight, preferably present within the formulation at an amount of 0.1 - 1 % in weight compared to the total weight of the formulation.

In one embodiment, the anti-microbial agent is non-covalently bonded to the resultant polymerized material. The anti-microbial agent is considered to be retained with the resultant polymerized material by non-covalent bonding, thus resulting in prolonged retention of the anti-microbial agent. In one embodiment, the non-covalent bonding allows the anti-microbial agent to be slowly released from the resultant polymerized material thereby enabling the polymerized material to exhibit antimicrobial properties on the surface over a prolonged period of time. The non-covalently bonded anti-microbial agent has been found to be retained within the polymerized film up to and including 2 hours, preferably retained within the polymerized film up to and including 8 hours, preferably retained within the polymerized film up to and including 24 hours, preferably up to and including 72 hours, preferably up to and including 1 week, preferably up to and including 1 month, preferably up to and including 1 year.

In one embodiment, the anti-microbial agent is covalently bonded to the resultant polymerized material.

The anti-microbial agent may for example comprise a modified anti-microbial agent, in which the modified anti-microbial agent comprises one or more reactive group for covalent bonding with the polymerizable material and/or polymerized material during polymerisation (for example photopolymerization on exposure to ambient light) to covalently anchor the anti-microbial agent in position.

It is important that the type of reactive group and the location of the reactive group(s) on the anti-microbial agent is selected to ensure that the or each reactive group does not affect the antimicrobial activity of the anti-microbial agent.

In one embodiment, the anti-microbial agent has at least one tether, in the form of for example a long alkyl chain, upon which one or more reactive group can be positioned for covalent bonding to the polymerizable material and/or polymerized material. The reactive group is preferably a radically polymerizable group.

For example, in one embodiment, the modified anti-microbial agent has the Formula (V):

A-B-C (V)

wherein:
Preferably A is the radically polymerizable group.
   Preferably A is the radically polymerizable group selected from the group consisting of: acrylate; methacrylate; acrylamide; methacrylamide; but-3-en-2-one; inverse methacrylate of formula 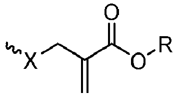 where R is an alkyl, X = O, NH; vinylsulfone; vinylurea; vinylcarbonate; vinylcarbamate; vinylthioester; vinylthiourea.
   Preferably A is the radically polymerizable group selected from the group consisting of: acrylate; methacrylate; acrylamide; methacrylamide; but-3-en-2-one; inverse methacrylate of formula 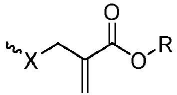 where R is an alkyl, X = O, NH; vinylsulfone.
   Preferably A is the radically polymerizable group selected from the group consisting of: acrylate; methacrylate; acrylamide; methacrylamide.
   Preferably A is the radically polymerizable group selected from the group consisting of: acrylate; methacrylate.
B is a linker selected among: C₂ to C₅₀ linear or branched alkyl chains, oligo(ethylene glycol): 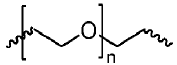 with n comprising between 1 to 3.
   Preferably B is a linker selected among C₂ to C₂₀ linear or branched alkyl chains, oligo(ethylene glycol): 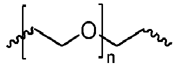 with n comprising between 1 to 3.
   Preferably B is a linker selected among C₅ to C₂₀ linear alkyl chains, oligo(ethylene glycol): 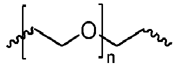 with n comprising between 1 to 3; and
C is a nitrogen-containing salt selected from the group consisting of:
   i) a group having the formula 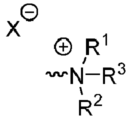
      where R¹, R² and R³ can be equal or different, and are each selected from the group comprising:
      benzyl, 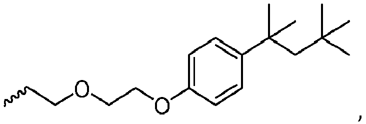 C₁ to C₅₀ linear or branched chains; and
      X is selected from the group comprising: halide, sulfate, carbonate or hydroxide.
      or
   ii) a group having the formula: 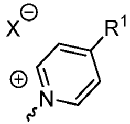
      where R¹ is selected among C₁ to C₅₀ linear or branched chains; and
      X is selected from the group comprising: halide, sulfate, carbonate or hydroxide.

Preferably, C is a nitrogen-containig salt selected from the group consisting of:
i) a group having the formula: 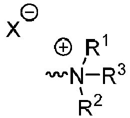
   where R¹, R² and R³ can be equal or different and are selected among benzyl, 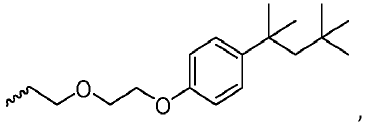 C₁ to C₂₀ linear or branched chains; and
   X is selected from the group comprising: halide, sulfate, carbonate or hydroxide;
   or
ii) a group having the formula: 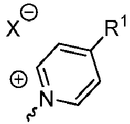
   where R¹ is selected among C₁ to C₂₀ linear or branched chains; and
   X is selected from the group comprising: halide, sulfate, carbonate or hydroxide.

Preferably the C group is a nitrogen-containing salt selected from the group consisting of:
i) a group having the formula 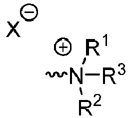
   where R¹, R² and R³ can be equal or different and are selected among benzyl, 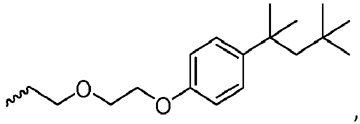 C₁ to C₂₀ linear chains;
   and X is selected from the group comprising halide, sulfate, carbonate or hydroxide; or
ii) a group having the formula 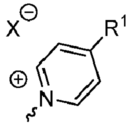
   where R¹ is selected among C₁ to C₂₀ linear chains; and
   X is selected from the group comprising halide, sulfate, carbonate or hydroxide.

For example, a modified anti-microbial agent is prepared by esterification of *N,N-*benzylmethylamino-2-ethanol with methacryloyl chloride. The ensuing ester is then reacted with an alkyl bromide to deliver the desired anti-microbial alkyl ammonium salt that is also capable of polymerization (Scheme I).

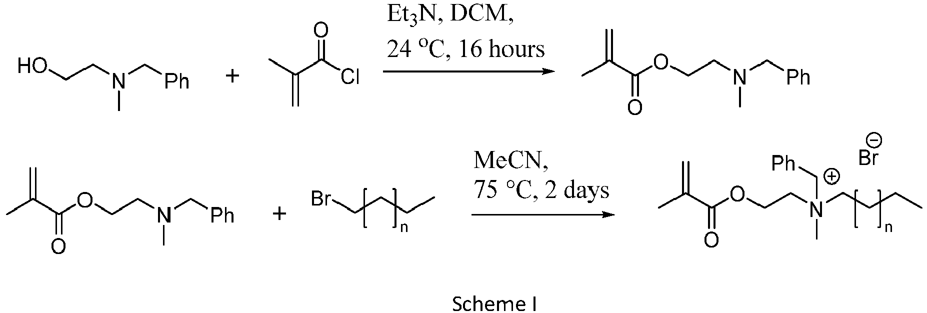

It is to be understood that the formulation may comprise any suitable number of anti-microbial agents, in any suitable combination, depending on the requirements for the formulation.

Covalent bonding of the anti-microbial agent within the polymerized material provides for enhanced retention of the anti-microbial agent on the contact surface. It has been found that polymerized material formed from the formulation of the present invention, comprising at least one anti-microbial agent covalently bonded to the material, provides anti-microbial activity for a period of at least 2 hours when immersed in water and remains anti-microbially active after 20 washes. It has been found that the polymerizable sanitizing formulation (preferably the photopolymerizable sanitizing formulation), in particular the resultant polymerized anti-microbial agent retaining material (for example the hydrogel polymers), of the present invention are able to be retained in place on a contact surface, preferably a user's skin, with an improved lifespan compared to conventional sanitizing formulations. The polymerizable sanitizing formulation (preferably the photopolymerizable sanitizing formulation), in particular the resultant polymerized anti-microbial agent retaining material (for example the hydrogel polymers), of the present invention have been found to show improved resistance against water rinsing, and being removed by for example sweat produced by the pores of a user, compared to conventional sanitizing formulations. The polymerizable sanitizing formulation (preferably the photopolymerizable sanitizing formulation), in particular the resultant polymerized anti-microbial agent retaining material (for example the hydrogel polymers), of the present invention have therefore been found to provide a longer lasting antimicrobial effect than conventional sanitizing formulations.

According to a second aspect of the present invention, there is provided a method for producing a polymerizable sanitizing formulation (preferably the photopolymerizable sanitizing formulation) for forming a polymerized anti-microbial agent retaining material on a contact surface, the method comprising:
obtaining a polymerizable water-soluble material;
optionally obtaining a non-polymerizable water-soluble material comprising PEG, PPG, or copolymer moieties thereof;
obtaining at least one initiator (preferably at least one photo-initiator) for radical polymerization of the polymerizable material to form a polymerized anti-microbial agent retaining material;
obtaining at least one anti-microbial agent for retention within the polymerized anti-microbial agent retaining material;
optionally obtaining at least one coinitiator to assist the initiator in radical polymerization of the polymerizable material;
optionally obtaining at least one antioxidant; and
obtaining at least one solvent.
mixing the polymerizable water soluble material, optionally the non-polymerizable water soluble material, at least one anti-microbial agent, optionally at least one antioxidant, and at least one solvent together and adding at least one initiator (preferably at least one photo-initiator) to provide a polymerizable sanitizing formulation.

The method of formation of the polymerized anti-microbial agent retaining material may use any conventional techniques for polymerizing the polymerizable water-soluble material.

The method may involve mixing the polymerizable water-soluble material (and optionally non-polymerizable water-soluble material) with an anti-microbial agent (optionally together with one or more coinitiator(s) and/or antioxidant(s)) to form a first mixture. The first mixture may be heated to a predetermined elevated temperature, for example to a temperature of at least 30 °C, preferably at least 40 °C, for example about 50 °C. The first mixture may be agitated, for example by stirring. At least one initiator may be present within the first mixture or subsequently added to the first mixture to form a second mixture, which may be agitated (for example by stirring) and/or heated to or maintained at a predetermined elevated temperature, for example to a temperature of at least 30 °C, preferably at least 40 °C, for example about 50 °C.

For example, in one embodiment, a method of formation of a polymerized anti-microbial agent retaining material (Formula III) from the polymerizable material comprising PEG-PPG copolymers (Formula II) is shown below:

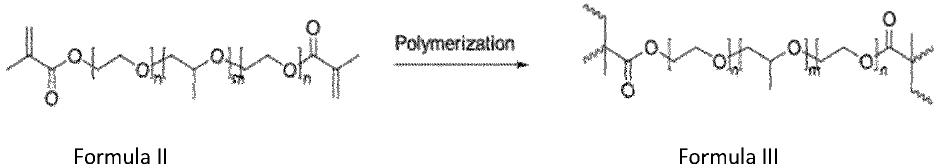

For the production of the polymerized anti-microbial agent (Formulation III), polymerizable water-soluble material comprising PEG, PPG, or copolymer moieties thereof is added to a suitable solvent. Afterwards, at least one antimicrobial agent (optional together with coinitiators and/or optional antioxidant) is added in suitable solvents to form a first mixture. The first mixture is then heated to a predetermined temperature and agitated for a predetermined time period. For example, the mixture may be placed into a thermoshaker and heated to 50°C and shaken (1200 rpm) for 20 minutes. The initiator(s) (for example the photoinitiator(s), are added to the first mixture in a suitable solvent to form a second mixture and well mixed at room temperature for a further predetermined time period, for example for 20 seconds.

The second mixture is then spread on a prewashed contact surface (for example pig skin/glass) and exposed to a light source, for example by placing into a LED-chamber. Afterwards, the curing grade as well as the properties of the polymerized anti-microbial agent retaining material (for example polymerized anti-microbial retaining hydrogel polymer) (Formula III) are assessed.

In one embodiment, the amount of light provided by the light source to the second mixture may be in the range of from 10 - 100000 lumen (blue/green/red - LED), preferably between 100 - 50000 lumen (blue/green/red - LED), preferably between 200 - 10000 lumen (blue/green/red - LED), preferably between 300 - 5000 lumen (blue/green/red - LED), preferably between 500 - 2000 lumen, blue/green/red - LED) for a predetermined time period, for example for a time period of from 5 seconds up to 60 minutes, preferably from 10 seconds to 30 minutes, preferably from 15 seconds to 15 minutes, preferably from 20 seconds to 10 minutes, preferably from 25 seconds to 5 minutes, for example from 30 seconds to 2 minutes.

According to a further aspect, the present invention provides the use of a polymerizable sanitisation formulation (preferably a photopolymerizable sanitisation formulation) as herein described for the formation of a polymerized anti-microbial agent retaining material on a contact surface.

According to a further aspect, the present invention provides the use of a polymerizable sanitisation formulation as herein described for sanitisation of a contact surface. The present invention preferably provides the use of a polymerizable sanitisation formulation as herein described for long lasting, sanitisation of a contact surface. In one embodiment, the present invention provides the use of a polymerizable sanitisation formulation as herein described for sanitisation, preferably long term sanitisation, of a contact surface against bilipid membrane containing microbe contamination, such as for example bacteria contamination, such as for example against Gram positive S. *aureus* and *Gram negative P. aeruginosa* contamination.

The term "long lasting" is used herein to refer to an effect, in this case a sanitisation effect, which is maintained over a period of time which is greater than or equal to 2 hours, preferably at least 8 hours, preferably at least 24 hours, preferably at least 72 hours, preferably at least 1 week, preferably at least 1 month, preferably at least 1 year.

The polymerizable sanitisation formulation of the present invention is preferably used to reduce and/or eliminate microbial contamination of a contact surface. The polymerizable sanitisation formulation of the present invention is preferably used to reduce and/or eliminate bacterial and/or viral and/or fungal infestations on a contact surface. The polymerizable sanitisation formulation of the present invention is preferably used to prevent an infection caused by microbial contamination of a contact surface.

The formulation may be applied to any suitable contact surface, including for example, but not to be limited to, hard surfaces such as kitchen surfaces and/or appliances; bathroom surfaces and/or appliances; floors; woodwork; metalwork; domestic appliances; industrial appliances; soft furnishings such as fabrics, textiles, laundry; skin and/or hair etc. Preferably the formulation is applied to skin. In one embodiment, the polymerizable sanitisation formulation (preferably the photopolymerizable sanitizing formulation) is a polymerizable hand sanitisation formulation (preferably a photopolymerizable hand sanitisation formulation).

The present invention provides a polymerizable sanitisation formulation (preferably a photopolymerizable sanitizing formulation) with improved retention of the anti-microbial agent at the site of application. As such, the formulation of the present invention could be used to reduce the risk of post-surgical infection. The photopolymerizable sanitisation formulation may however also be used in non-medical applications.

According to a further aspect of the present invention there is provided an apparatus for dispensing a polymerizable sanitizing formulation (preferably a photopolymerizable sanitizing formulation) as herein described, the apparatus comprising:
a reservoir comprising a polymerizable sanitizing formulation (preferably a photopolymerizable sanitizing formulation) comprising:
   a) a polymerizable water-soluble material;
   b) at least one initiator (preferably photo-initiator) for radical polymerization of the polymerizable water-soluble material to form a polymerized anti-microbial agent retaining material;
   c) at least one anti-microbial agent for retention within the polymerized anti-microbial agent retaining material; and
   d) a solvent; and
a dispensing nozzle in fluid communication with the reservoir and configured to dispense the polymerizable sanitizing formulation on to a contact surface.

The formulation may be applied to a contact surface using any suitable dispensing mechanism, such as for example by use of an aerosol, a spray, a fogging mechanism, a lotion, gel or ointment. The formulation is preferably stored within a sealed container which ensures the formulation is not exposed to light prior to use.

The polymerized anti-microbial agent retaining material is preferably provided as a film or coating on the contact surface.

### EXAMPLES

### Example 1 - Synthesis of a polvmerizable material

Poly(ethylene glycol) 2k (1 equiv) was loaded in a round-bottom flask coupled with a magnetic stirring bar and dissolved in dry dichloromethane (DCM, 0.1 M). Dry triethylamine (4 equiv) was added to the solution. Methacryloyl chloride (freshly distilled, 3.5 equiv) was then added at 0 °C and the reaction was left to stir at 25 °C for 14 hours in the dark. After completion was confirmed by H NMR, the reaction was quenched with water and extracted with DCM. The combined organic extracts were passed through a plug of basic alumina, which was eluted with DCM. The solvent was then removed under reduced pressure to a viscous oil and no further. The crude oily residue was vigorously stirred, and diethyl ether was then added until a substantial precipitate was observed. The solution was then further cooled in an ice bath to maximize the yield of precipitate. The solid was collected via vacuum filtration over a frit, washed with diethyl ether, and was further dried under high vacuum to yield the product as a white solid. ¹H NMR (CDCl₃, 300 MHz) δ (ppm): 6.10 (s, 2H, vinyl), 5.54 (s, 2H, vinyl), 4.35 - 4.17 (m, 4H, CO₂CH₂), 3.77 - 3.66 (m, 4H), 3.61 (br s, PEG), 1.92 (s, 6H, CH₃).

### Example 2 - Synthesis of a polymerizable material

Poly(ethylene glycol) 6k (1 equiv) and triethylamine (3 equiv) were added to dry DCM (0.17 M) in a roundbottomed flask equipped with a magnetic stir bar. Methacryloyl chloride (freshly distilled, 2.6 equiv) was then added at 0 °C and the reaction was left to stir at 25 °C for 14 hours in the dark. Once complete the mixture was passed through a column of basic alumina using DCM as eluent. The filtrate was concentrated under reduced pressure to approximately 5% volume. The resulting crude oily product was added to rapidly stirring diethyl ether at room temperature, which was stirred for 30 minutes. The mixture was then cooled to 0 °C and stirred for a further 30 minutes. The precipitate was collected via vacuum filtration over a frit, washed with diethyl ether, and was dried under high vacuum to yield the product, poly(ethylene glycol) dimethacrylate 6k, as a white solid. ¹H NMR (DMSO-d₆, 300 MHz) δ (ppm): 6.03 (t, J = 1.2 Hz, 2H, vinyl), 5.69 (t, J = 1.6 Hz, 2H, vinyl), 4.22 - 4.19 (m, 4H, CH2), 3.67 - 3.63 (m, 4H, CH2), 3.51 (s, PEG), 1.88 (t, J = 1.1 Hz, 3H, CH3).

### Example 3 - Synthesis of a polymerizable material

Poly(ethylene glycol) 20k (1 equiv) was loaded in a round bottom flask. Dry DCM was added until the solution became clear (0.08 M). Triethylamine (2.20 equiv) and methacrylic anhydride (3 equiv) were added, the reaction flask was covered with aluminium foil and the solution was stirred for 5 days at 20 °C in the dark. The solution was filtered through a 5 cm plug of neutral aluminium oxide to remove the methacrylic acid by-product. Subsequently, about 150 mL of solvent was removed under reduced pressure, the product precipitated by addition of diethyl ether, collected on a large G3 glass frit, washed with diethyl ether and dried under reduced pressure, to obtain poly(ethylene glycol) dimethacrylate 20k as a fine, white powder. ¹H NMR (CDCl₃, 300 MHz) δ 6.04 (s, 2H, vinyl), 5.49 (s, 2H, vinyl), 4.28-4.11 (m, 4H, CH₂), 3.56 (br s, PEG), 1.86 (s, 6H, CH₃).

### Example 4 - Synthesis of a polvmerizable material

Poly(ethylene glycol) 2k (1 equiv) was loaded into a round-bottom flask coupled with a magnetic stirring bar. Dichloromethane (0.17 M) and triethylamine (3 equiv) were added and the solution was cooled down to 0 °C. Methanesulfonyl chloride (3 equiv) was then added slowly and the reaction was left to stir at 0 °C for 45 min and was warmed to room temperature overnight. Once complete, the reaction was quenched with water and extracted with DCM. The aqueous phase was washed with DCM. The combined organic layer was dried over magnesium sulfate. The mixture was filtered, and the filtrate was concentrated under reduced pressure to approximately 5 % volume. The resulting crude oily product was added to rapidly stirring diethyl ether at room temperature, which was stirred for 30 minutes. The mixture was then cooled to 0 °C and stirred for a further 30 minutes. The precipitate was collected via vacuum filtration over a frit, washed with diethyl ether, and was dried under high vacuum to yield the poly(ethylene glycol) dimesylate 2k as a white solid. ¹H NMR (DMSO-d₆, 300 MHz) δ (ppm): 4.34-4.27 (m, 4H, MsOC*H₂*), 3.72-3.63 (m, 4H, CH₂), 3.51 (br s, PEG), 3.17 (s, 6H, CH₃).

Poly(ethylene glycol) dimesylate 2k was transferred into a round-bottom flask coupled with a magnetic stirring bar. Aqueous ammonia solution (25 %, approx. 5 mL/mmol) was added into the flask, which was tightly sealed with a stopper and metal clamp. The reaction was vigorously stirred for approximately. 3 days. Once full conversion was reached, the aqueous layer was extracted four times with dichloromethane and the combined organic extracts were dried over magnesium sulfate and concentrated under reduced pressure but only to a viscous oil. The remaining residue was vigorously stirred, and diethyl ether was then added until a substantial precipitate was observed. The solution was further cooled down in an ice bath to maximize the precipitate yield. The precipitate was collected via vacuum filtration over a frit, washed with diethyl ether, and was further dried under high vacuum to yield poly(ethylene glycol) diamine 2k as a white solid. ¹H NMR (DMSO-d₆, 300 MHz) δ (ppm): 3.51 (br s, PEG), 3.36 (t, J = 5.8 Hz, 4H, CH₂), 2.66 (t, J = 5.7 Hz, 4H, CH₂).

Poly(ethylene glycol) diamine 2k (1 equiv) was loaded into a round-bottom flask coupled with a magnetic stirring bar and dissolved in dichloromethane (0.18 M). Acryloyl chloride (distilled, 3.3 equiv) was added followed by sodium hydroxide solution (1 M, 3.2 equiv). The biphasic solution was vigorously stirred at 25 °C for 1.5 h. After the reaction reached completion, sodium hydroxide solution (1 M, 3.2 equiv) was added and the mixture was stirred for 5 min. The mixture was transferred into a separatory funnel and the aqueous layer was extracted with DCM. The organic phase was passed through a plug of basic alumina. The plug was washed with DCM as eluent. The solvent was then removed under reduced pressure but only to a viscous oil. The remaining residue was vigorously stirred. Diethyl ether was then added until more noticeable precipitation was observed. The solution was further cooled down in an ice bath to maximize the precipitate yield. The precipitate was collected via vacuum filtration over a frit, washed with diethyl ether. The white powder was dried under high vacuum to remove the ether and was then lyophilized to obtain poly(ethylene glycol) diacrylamide 2k. ¹H NMR (CDCl₃, 300 MHz) δ (ppm): 6.54 (br s, 2H, NH), 6.28 (dd, J = 1.5, 17.0 Hz, 2H, vinyl), 6.14 (dd, J = 10.0, 17.0 Hz, 2H, vinyl), 5.61 (d, J = 10.0 Hz, 2H, vinyl), 3.63 (br s, PEG), 3.52 (dd, J = 5.0, 10.1 Hz, 4H, CH₂).

### Example 5 - Synthesis of a polymerizable material

Pluronic^{®} F68 (1 equiv) was loaded in a round-bottom flask coupled with a magnetic stirring bar and dissolved in dry DCM (0.1 M). Dry triethylamine (4 equiv) was added to the solution. Methacryloyl chloride (freshly distilled, 3.5 equiv) was then added at 0 °C and the reaction was left to stir at 25 °C for 14 h in the dark. After completion, the reaction was quenched with water and extracted with DCM. The combined organic extracts were passed through a plug of basic alumina, which was eluted with DCM. The solvent was then removed under reduced. pressure to a viscous oil. The crude oily residue was dissolved in water and was dialyzed in Spectra/Por 6 membrane tubing against water for 24 hours. The dialyzed solution was then lyophilized to dryness to yield the product F68 dimethacrylate as a viscous colourless oil. ¹H NMR (DMSO-d₆, 300 MHz) δ (ppm): 6.03 (s, 2H, vinyl), 5.69 (s, 2H, vinyl), 4.20 (t, J = 4.7 Hz, 4H, OCH₂), 3.50 (s, backbone), 1.88 (s, 6H, CH₃), 1.03 (d, J = 5.5 Hz,CH₃ backbone).

### Example 6 - Synthesis of a polymerizable material

Tetronic^{®} 1107 (1 equiv) was loaded in a round-bottom flask coupled with a magnetic stirring bar and dissolved in dry dichloromethane (0.1 M). Dry triethylamine (8.5 equiv) was added to the solution. Methacryloyl chloride (freshly distilled, 7.5 equiv) was then added at 0 °C and the reaction was left to stir at 25 °C for 48 h in the dark. After completion was confirmed by H NMR, the reaction was quenched with water and extracted with DCM. The combined organic extracts were passed through a plug of basic alumina, which was eluted with DCM. The solvent was then removed under reduced pressure to a viscous oil and no further. The remaining oil was vigorously stirred, and diethyl ether was added until a substantial precipitate was observed. Mixture was then placed in ice to maximize the yield of precipitate. The solid was collected via vacuum filtration over a frit, washed with cold diethyl ether, and was further dried under high vacuum to yield the product Tetronic 1107 tetramethacrylate as a white solid. ¹H NMR (DMSO-d₆, 300 MHz) δ (ppm): 6.03 (s, 4H, vinyl), 5.69 (s, 4H, vinyl), 4.25-4.17 (m, 8H, CO₂CH₂), 3.90-3.81 (m, 8H), 3.51 (br s, PEG), 3.33 (br s, PPG), 1.88 (s, 12H, CH₃), 1.03 (d, CH₃ on the PPG).

### Example 7 - Synthesis of a polymerizable material

Inverse Pluronic^{®} 10R5 (1 equiv) was loaded in a round-bottom flask coupled with a magnetic stirring bar and dissolved in dry DCM (0.1 M). Dry triethylamine (4 equiv) was added to the solution. Methacryloyl chloride (freshly distilled, 3.5 equiv) was then added at 0 °C and the reaction was left to stir at 25 °C for 14 h in the dark. After completion, the reaction was quenched with water and extracted with DCM. The combined organic extracts were passed through a plug of basic alumina, which was eluted with DCM. The solvent was then removed under reduced. pressure to a viscous oil. The crude oily residue was dissolved in water and was dialyzed in Spectra/Por 6 membrane tubing against water for 24 hours. The dialyzed solution was then lyophilized to dryness to yield the product 10R5 dimethacrylate as a viscous colourless oil. ¹H NMR (CDCl₃, 300 MHz) δ (ppm): 6.08 (dd, J = 1.0, 1.8 Hz, 2H, vinyl), 5.53-5.52 (m, 2H, vinyl), 5.09-5.02 (m, 1H, CH), 3.63-3.36 (m, PEG and PPG), 3.41 (t, J = 6.5 Hz, 4H, 5 CH2), 1.92 (m, 6H, CH3), 1.26-1.23 (m, 6H, CH₃), 1.13-1.11 (m, PPG).

### Example 8 - Synthesis of a polymerizable material

To the poly(ethylene glycol) 2k (1 equiv) dissolved in dry tetrahydrofuran (THF, 0.01 M), sodium hydride (10 equiv, 60% dispersion in mineral oil) was added under argon. After hydrogen evolution, divinyl sulfone (10 equiv) was added very quickly. The reaction was carried out at 25°C for 48 hours under an argon atmosphere with constant stirring. Afterward, the reaction solution was neutralized with concentrated acetic acid, filtered until clear, and reduced to a small volume by rotary evaporation. The crude product was precipitated by adding the remaining solution dropwise into ice-cold diethyl ether. The polymer was recovered by filtration, washed with diethyl ether, and dried under vacuum. The dry polymer was then dissolved in water containing sodium chloride and extracted 3 times with dichloromethane. This solution was dried with magnesium sulfate, filtrated, and the volume was reduced by rotary evaporation. Finally, poly(ethylene glycol) di(vinyl sulfone) was precipitated and thoroughly washed with diethyl ether to remove all remaining divinyl sulfone. ¹H NMR (CDCl₃, 300 MHz) δ 6.76 (dd, J = 16.6, 9.9 Hz, 2H, CH), 6.31 (d, J = 16.6 Hz, 2H, vinyl), 6.02 (d, J = 9.9 Hz, 2H, vinyl), 3.82 (t, J = 5.7 Hz, 4H, OCH₂), 3.57 (s, PEG), 3.18 (t, J = 5.6 Hz, 4H, SCH₂).

### Example 9 - Synthesis of an antimicrobial agent

1-Chloro-2-(2-chloroethoxy)ethane (6 equiv) was added dropwise to a mixture of potassium carbonate (3 equiv) and 4-(2,4,4-trimethylpentan-2-yl)phenol (1 equiv) in dry dimethylformamide (1.5 M) at 80 °C. The reaction was stirred for 14 hours, then potassium carbonate was removed via filtration, and the solution was concentrated *in vacuo* to obtain a liquid in which toluene was added. The solution was concentrated via rotary evaporation to remove the excess 1-chloro-2-(2-chloroethoxy)ethane to then obtain the product 1-(2-(2-chloroethoxy)ethoxy)-4-(2,4,4-trimethylpentan-2-yl)benzene as a yellow liquid. ¹H NMR (CDCl₃, 300 MHz) δ 7.24 (d, J = 3.2 Hz, 2H, CH), 6.81 (d, J = 8.8 Hz, 2H, CH), 4.11 (dd, J = 5.7, 3.9 Hz, 2H, CH₂), 3.90 - 3.76 (m, 4H, CH₂), 3.64 (t, J = 5.9 Hz, 2H, CH₂), 1.68 (s, 2H, CH₂), 1.32 (s, 6H, CH₃), 0.69 (s, 9H, CH₃).

Methacrylic acid 2-(dimethylamino)ethyl ester (1.1 equiv) and 1-(2-(2-chloroethoxy)ethoxy)-4-(2,4,4-trimethylpentan-2-yl)benzene (1 equiv) were dissolved in acetonitrile (0.5 M). The mixture was purged with nitrogen gas for 20 min and stirred for 4 days at 65 °C. The solution was concentrated *in vacuo* to obtain an oil which was vigorously stirred and diethyl ether was added until a precipitate was observed. The mixture was cooled to 0 °C to maximize precipitation and stirred for 20 min. The precipitate was collected, washed with diethyl ether and dried under high vacuum to obtain the product 2-(methacryloyloxy)-*N,N*-dimethyl-*N*-(2-(2-(4-(2,4,4-trimethylpentan-2-yl)phenoxy)ethoxy)ethyl)ethan-1-aminium chloride as a white solid. ¹H NMR (CDCl₃, 300 MHz) δ 7.25 (d, J = 3.1 Hz, 2H, CH), 6.81 - 6.72 (m, 2H, CH), 6.16 - 6.07 (m, 1H, vinyl), 5.67 - 5.58 (m, 1H, vinyl), 4.62 (s, 2H, CH₂), 4.20 - 4.04 (m, 8H, CH₂), 3.92 - 3.82 (m, 2H, CH₂), 3.51 (s, 6H, CH₃), 1.93 (t, J = 1.3 Hz, 3H, CH₃), 1.69 (s, 2H, CH₂), 1.33 (s, 6H, CH₃), 0.70 (s, 9H, CH₃).

### Example 10 - Synthesis of an antimicrobial agent

A round-bottom flask was charged with 16-bromo-1-hexadecanol (1 equiv) and pyridine (0.3 M), and stirred for 48 hours at 60 °C. After the reaction was completed the mixture was cooled to 25 °C, evaporated under *vacuo* until a white solid precipitated appeared. The white solid was filtered, washed with cold diethyl ether, and dried under high vacuum to give the pyridinium salt 1-(16-hydroxyhexadecyl)pyridin-1-ium. This was used without further purification in the next step.

1-(16-hydroxyhexadecyl)pyridin-1-ium was dissolved in chloroform (0.1 M). After cooling the solution to 0 °C, methacryloyl chloride (2.5 equiv) was added dropwise via a syringe. The reaction mixture was stirred first at 0 °C for 30 min and then at 45 °C for 48 hours, protected with aluminium foil. After the reaction was completed the solution was poured into a separation funnel and the organic layer was washed with aqueous solution of sodium bicarbonate, water and brine, dried over anhydrous sodium sulfate, filtered and concentrated under reduced pressure *in vacuo.* Removal of the solvent gives the product as a yellowish oil ¹H NMR (CDCl₃, 300 MHz) δ (ppm): 9.45 (m, 2H, Py), 8.47 (m, 1H, Py), 8.10 (m, 2H, Py), 6.09 (s, 1H, vinyl), 5.55 (s, 1H, vinyl), 5.03 (t, 2H, -CH₂N), 4.13 (t, 2H, -CO₂CH₂), 1.16-2.12 (m, 28H hydrocarbon chain), 1.94 (s, 3H, CH₃).

### Example 11 - Synthesis of an antimicrobial agent

*N*-Benzyl-*N*-methylethanolamine (1 equiv) was loaded in a round-bottom flask coupled with a magnetic stirring bar and dissolved in dichloromethane (3.0 M), and triethylamine (2 equiv) was added. The reaction mixture was cooled to 0 °C and methacryloyl chloride (freshly distilled, 1.5 equiv) was added and the reaction was stirred for 14 hours at 25 °C. The solution was diluted with dichloromethane and washed with water. The organic layers were dried over magnesium sulfate, passed through a plug of basic alumina, and dried under high vacuum to obtain 2-(benzylmethylamino)ethyl methacrylate as a light yellow liquid. ¹H NMR (CDCl₃, 300 MHz) δ 7.26-7.10 (m, 5H, ArH), 6.05 (dd, J = 1.8, 0.9 Hz, 1H, vinyl), 5.50 (t, J = 1.6 Hz, 1H, vinyl), 4.22 (t, J = 5.9 Hz, 2H, OCH₂), 3.51 (s, 2H, NCH₂), 2.65 (t, J = 5.9 Hz, 2H, NCH₂), 2.24 (s, 3H, CH₃), 1.93 - 1.84 (m, 3H, CH₃).

2-(Benzylmethylamino)ethyl methacrylate (1.1 equiv) and 1-bromododecane (1 equiv) were dissolved in acetonitrile (1.2 M). The mixture was purged with nitrogen gas for 20 min and stirred for 3 days at 75 °C. The solution was concentrated *in vacuo* to obtain an oil which was purified by column chromatography (Et₂O, 100% then DCM:MeOH, 90%) to obtain the product 2-(N,N,N-dodecylmethylbenzylammonium)ethyl methacrylate bromide as a white gel. ¹H NMR (CDCl₃, 300 MHz) δ 7.73 - 7.65 (m, 2H, ArH), 7.47 (dt, J = 10.4, 3.7 Hz, 3H, ArH), 6.18 - 6.09 (m, 1H, vinyl), 5.71 - 5.63 (m, 1H, vinyl), 5.33 - 4.95 (m, 2H, OCH₂), 4.73 - 4.62 (m, 2H, NCH₂), 4.28 - 3.93 (m, 2H, NCH₂), 3.51 - 3.46 (m, 3H, NCH₂), 3.35 - 3.28 (m, 2H, NCH₂), 1.95 (t, J = 1.3 Hz, 3H, CH₃), 1.91 - 1.69 (m, 2H, CH₂), 1.25 (s, 18H, CH₂), 0.96-0.79 (m, 3H, CH₃).

### Example 12- Formulation preparation and mechanical test

The sanitising gel sample was prepared by the addition of poly(ethylene glycol) dimethacrylate 6k (12.64 % w/w), prepared according to Example 2, to glycerol (29.48 % w/w). Then triethanolamine (0.84 % w/w) was added in water (7.58 % w/w), diphenyliodonium hexafluorophosphate (0.067 % w/w) and 4-(diphenylphosphino)benzoic acid (0.166 % w/w) were added in ethanol (8.15 % w/w) and didecyldimethylammonium chloride (1.05 % w/w) was added in PBS buffer x1 (40.01 % w/w). The mixture was placed into a thermoshaker and heated and shaken (50 °C, 1200 rpm) for 20 minutes. Next, eosin Y (0.012 % w/w) was added to the mixture in ethanol (1.23 % w/w) and was mixed at room temperature for 20 seconds.

0.5 mL of the formulation was then spread on a prewashed 4 cm² glass surface and photopolymerized in 1 minute using a LED-chamber (10000 lumens, white - LED). A thin polymer film was obtained.

### Example 13- Formulation preparation and mechanical test

The sanitising gel sample was prepared by the addition of Pluronic^{®} F68 dimethacrylate (12.85 % w/w), prepared according to Example 5, to glycerol (29.98 % w/w). Then triethanolamine (0.85 % w/w) was added in water (7.71 % w/w), diphenyliodonium hexafluorophosphate (0.068 % w/w) was added in ethanol (6.77 % w/w) and 2-(N,N,N-dodecylmethylbenzylammonium)ethyl methacrylate bromide (1.07 % w/w) prepared according to Example 11 was added in PBS buffer x1 (40.70 % w/w). The mixture was placed into a thermoshaker and heated and shaken (50 °C, 1200 rpm) for 20 minutes. Next, Irgacure^{®} 819 (0.31 % w/w) was added to the mixture in ethanol (2.81 % w/w) and was mixed at room temperature for 20 seconds.

0.5 mL of the formulation was then spread on a prewashed 4 cm² glass surface and photopolymerized in 30 seconds using a LED-chamber (10000 lumens, white - LED). A thin polymer film was obtained.

### Example 14- Formulation preparation and mechanical test

The sanitising gel sample was prepared by the addition of Tetronic^{®} 1107 tetramethacrylate (12.64 % w/w), prepared according to Example 6, to glycerol (29.48 % w/w). Then triethanolamine (0.84 % w/w) was added in water (7.58 % w/w), diphenyliodonium hexafluorophosphate (0.067 % w/w) and 4-(diphenylphosphino)benzoic acid (0.166 % w/w) were added in ethanol (8.16 % w/w) and 1-(16-(methacryloyloxy)hexadecyl)pyridin-1-ium bromide (1.05 % w/w), prepared according to Example 10, was added in PBS buffer x1 (40.01 % w/w). The mixture was placed into a thermoshaker and heated and shaken (50 °C, 1200 rpm) for 20 minutes. Next, ethyl (2,4,6-trimethylbenzoyl) phenylphosphinate (0.31 % w/w) and eosin Y (0.00125 % w/w) were added to the mixture in ethanol (4.01 % w/w) and the solution was mixed at room temperature for 20 seconds.

0.5 mL of the formulation was spread on a prewashed 4 cm² glass surface and polymerized in 30 minutes under ambient light. A thin polymer film was obtained.

### Example 15- Formulation preparation and mechanical test

The sanitising gel sample was prepared by the addition of Tetronic^{®} 1107 tetramethacrylate (12.64 % w/w), prepared according to Example 6, to glycerol (29.48 % w/w). Then triethanolamine (0.84 % w/w) was added in water (7.58 % w/w), diphenyliodonium hexafluorophosphate (0.067 % w/w) and 4-(diphenylphosphino)benzoic acid (0.17 % w/w) were added in ethanol (8.16 % w/w) The mixture was placed into a thermoshaker and heated and shaken (50 °C, 1200 rpm) for 20 minutes. Next, methylene blue (0.006 % w/w) was added to the mixture in ethanol (0.58 % w/w) and the solution mixed at room temperature for 20 seconds.

0.5 mL of the formulation was then spread on a prewashed 4 cm² glass surface and polymerized in 30 minutes using LED-chamber (5000 lumens, red - LED. A thin polymer film was obtained.

### Example 16- Formulation preparation and mechanical test

The sanitising gel sample was prepared by the addition of poly(ethylene glycol) diacrylate 700 (16.63 % w/w) to Pluronic^{®} PE 6400 (11.15 % w/w) and glycerol (43.64 % w/w). Then triethanolamine (1.57 % w/w) and 9-(N,N,N-benzylbutylmethylammonium)nonyl methacrylate bromide (0.05 % w/w) were added in water (23.83 % w/w), diphenyliodonium hexafluorophosphate (0.031 % w/w) was added in ethanol (3.10 % w/w). The mixture was placed into a thermoshaker and heated and shaken (50 °C, 1200 rpm) for 20 minutes. Next, rose bengal (0.00046 % w/w) was added to the mixture in ethanol (0.23 % w/w) and the solution was mixed at room temperature for 20 seconds.

0.5 mL of the formulation was then spread on a 4 cm² prewashed pig skin sample and photopolymerized in 1 minute using LED-chamber (10000 lumens, white - LED). A thin polymer film was obtained. The hydrogel on the pig skin was then inspected under a microscope. The pig skin was then stretched and folded. The hydrogel was then inspected under a microscope. No tears or breaks were observed in the film.

### Example 17- Formulation preparation and mechanical test

The sanitising gel sample was prepared by the addition of Tetronic^{®} 1107 tetramethacrylate (12.61 % w/w), prepared according to Example 6, to Pluronic^{®} PE 6400 (8.34 % w/w), tris(3-sulfonatophenyl)phosphine hydrate sodium salt (2.01 % w/w) and glycerol (30.95 % w/w). Then triethanolamine (0.9 % w/w) was added in water (8.11 % w/w), diphenyliodonium hexafluorophosphate (0.032 % w/w) and 16-(N,N,N-butyldimethylammonium)cetyl methacrylate bromide (0.040 % w/w) were added in ethanol (11.07 % w/w). The mixture was placed into a thermoshaker and heated and shaken (50 °C, 1200 rpm) for 20 minutes. Next, eosin Y (0.013 % w/w) was added to the mixture in ethanol (1.32 % w/w) and the solution was mixed at room temperature for 20 seconds.

0.5 mL of the formulation was then spread on a 4 cm² prewashed pig skin sample and photopolymerized in 1 minute using LED-chamber (10000 lumens, white - LED). A thin polymer film was obtained. The hydrogel on the pig skin was then inspected under a microscope. The pig skin was then stretched and folded. The hydrogel was then inspected under a microscope. No tears or breaks were observed in the film.

### Example 18- Formulation preparation and mechanical test

The sanitising gel sample was prepared by the addition of poly(ethylene glycol) diacrylamide 2k ( 12.64 % w/w), prepared according to Example 4, to glycerol (29.48 % w/w). Then triethanolamine (0.84 % w/w) was added in water (7.58 % w/w), diphenyliodonium hexafluorophosphate (0.067 % w/w) and 4-(diphenylphosphino)benzoic acid (0.17 % w/w) were added in ethanol (8.16 % w/w) and benzethonium chloride (1.05 % w/w) was added in PBS buffer x1 (40.01 % w/w). The mixture was placed into a thermoshaker and heated and shaken (50 °C, 1200 rpm) for 20 minutes. Next, erythrosin B (0.012 % w/w) was added to the mixture in ethanol (1.23 % w/w) and the solution was mixed at room temperature for 20 seconds.

0.5 mL of the formulation was then spread on a prewashed glass 4 cm² and photopolymerized in 1 minute using LED-chamber (10000 lumens, white - LED). A thin polymer film was obtained.

### Example 19- Formulation preparation and mechanical test

The sanitising gel sample was prepared by the addition of Tetronic^{®} 1107 tetramethacrylate (12.63 % w/w), prepared according to Example 6, to glycerol (29.48 % w/w). Then triethanolamine (0.84 % w/w) was added in water (7.58 % w/w), diphenyliodonium hexafluorophosphate (0.067 % w/w) and 4-(diphenylphosphino)benzoic acid (0.17 % w/w) were added in ethanol (8.16 % w/w) in PBS buffer x1 (40.01 % w/w). The mixture was placed into a thermoshaker and heated and shaken (50 °C, 1200 rpm) for 20 minutes. Next, camphorquinone (0.17 % w/w) was added to the mixture in ethanol (1.49 % w/w) and well mixed at room temperature for 20 seconds.

0.5 mL of the formulation was then spread on a prewashed glass 4 cm² and photopolymerized in 1 minute using LED-chamber (2000 lumens, white - LED). A thin polymer film was obtained.

### Example 20- Formulation preparation and mechanical test

The sanitising gel sample was prepared by adding in a 2 mL Eppendorf, poly(ethylene glycol) dimethacrylate 6k (9.09 % w/w), prepared according to Example 2. Afterwards, water (60.61 % w/w) was added, then (benzyl(9-(methacryloyloxy)nonyl)(methyl)-I4-azaneyl)methylium bromide (0.78 % w/w) was added in water (14.40 % w/w). The mixture was placed into a thermoshaker and heated and shaken (50 °C, 1200 rpm) for 10 minutes. Next, 2,2'-(azodiisopropylidene)di-2-imidazoline dihydrochloride (2.53 % w/w) was added to the mixture in water (12.63 % w/w) and the solution was mixed at room temperature for 20 seconds.

The Eppendorf was then warmed at 50 °C. Polymerization was determined by inverting the Eppendorf to see if the solution was liquid (flowing) or solid. After 8 minutes a soft polymeric material was observed.

### Example 21- Formulation preparation and mechanical test

The sanitising gel sample was prepared by the addition of poly(ethylene glycol) dimethacrylate 6k (10.00 % w/w), prepared according to Example 2, to glycerol (23.33 % w/w) and water (33.33 % w/w). Then (benzyl(9-(methacryloyloxy)nonyl)(methyl)-I4-azaneyl)methylium bromide (0.83 % w/w) was added in water (15.83 % w/w). The mixture was placed into a thermoshaker and heated and shaken (50 °C, 1200 rpm) for 10 minutes. Next, 2,2'-(azodiisopropylidene)di-2-imidazoline dihydrochloride (2.78 % w/w) was added to the mixture in water (13.88 % w/w) and the solution was mixed at room temperature for 10 seconds.

0.1 mL of the formulation was then spread on a prewashed glass 1 cm². The glass surface was placed on a 50 °C heating plate. Polymerization occur in less than 5 minutes.

### Example 22 - Biological preparation

The anti-microbial-retaining hydrogel formulation was prepared by the addition of Tetronic^{®} 1107 tetramethacrylate (12.64 % w/w), prepared according to Example 6, to glycerol (29.48 % w/w). Then triethanolamine (0.84 % w/w) was added in water (7.58 % w/w), diphenyliodonium hexafluorophosphate (0.067 % w/w) and 4-(diphenylphosphino)benzoic acid (0.166 % w/w) were added in ethanol (8.16 % w/w) and a specified amount of anti-microbial agent was added in PBS buffer x1 (40.01 % w/w). The mixture was placed into a thermoshaker and heated and shaken (50 °C, 1200 rpm) for 20 minutes. Next, ethyl (2,4,6-trimethylbenzoyl) phenylphosphinate (0.31 % w/w) and eosin Y (0.00125 % w/w) were added to the mixture in ethanol (4.01 % w/w) and the solution was mixed at room temperature for 20 seconds.

A 15 mL falcon tube containing 5 mL of BHI medium was inoculated with *Staphylococcus aureus* (ATCC 6538) and incubated overnight at 37 °C on a shaker (140 rpm). On the next day, a 15 mL falcon tube containing 5 mL BHI medium was inoculated with 100 µL of the overnight culture and incubated at 37 °C for 3.5 hours at 140 rpm.

Glass plates (18 mm x 18 mm) were added with anti-microbial-retaining hydrogel formulation (50 µL) and covered with another glass plate (18 mm x 18 mm) in the well of a 6-well plate. Samples were polymerized by the light from the laminar flow hood for 30 minutes. The upper glass plate was removed with a scalpel, and the gels washed 20 times with sterile distilled water (1 mL).

*S. aureus* cultures were diluted to an OD600 (optical density at λ=600 nm) of 0.2 and 50 µL added to the glass plates (18 mm x 18 mm) covered with the anti-microbial retaining hydrogel formulation of the present invention. Samples were covered with a glass plate (18 mm x 18 mm) and incubated at 37 °C for 60 minutes. Bacteria were recovered by addition of 1 mL of BHI medium to each well and manually shaking the plate for 30 seconds. From each solution a four times ten-fold dilution series was prepared, resulting in 5 concentrations (each time adding 20 µL of the previous solution to 180 µL of PBS). A BHI agar plate was divided into 5 sectors and from each dilution, three drops of 10 µL were added to one sector. The plates were incubated overnight at 37 °C. From the dilution containing 3-30 colonies, the CFU/mL (colony forming units per mL) were calculated and recorded.

Samples were prepared as triplicates. As negative controls, glass plates with no hydrogel and with hydrogel but no anti-microbial agent were prepared. As a control group, glass plates were added with stock solutions of the appropriate disinfectant in PBS (50 µL) at the same concentration as in the hydrogel.

For the washing of samples, the polymerized hydrogel sample was washed with 1 mL PBS solution, after which the PBS solution was removed. The sample was let air dry for 15 minutes before bacterial was then applied as described above.

### Example 23 - Biological test

Direct contact tests were carried out according to Example 22 with benzethonium chloride (0.1 % w/w) on a glass surface with no hydrogel as a control and benzethonium chloride (0.1 % w/w) in the polymerized hydrogel. The Log₁₀ reduction of *S. aureus* was then assessed after 0, 5, 10, and 20 washes with 1 mL water.

| **Surfaces/carrier** | **0 washes** | **5 washes** | **10 washes** | **20 washes** |
|---|---|---|---|---|
| Anti-microbial agent on glass surface | >4 | <1 | <1 | <1 |
| Anti-microbial agent retained hydrogel formulation | 2 | 3 | 3 | 2 |
| Hydrogel without anti-microbial agent | <1 | <1 | <1 | <1 |

### Example 24 - Biological test

Direct contact tests were carried out with hydrogel formulations according to Example 22 with unmodified anti-microbial agents (0.1 % w/w). The Log₁₀ reduction of *S. aureus* was then assessed after 0 and 5 washes with 1 mL water.

| **Anti-microbial agent** | **0 washes** | **5 washes** |
|---|---|---|
| Benzethonium chloride | 2 | 3 |
| Benzalkonium chloride | 2 | >4 |
| Chlorohexidine | >4 | >4 |
| Didecyldimethylammonium chloride | >3 | >4 |
| Sterilium with no hydrogel | >4 | 1 |
| Hydrogel without anti-microbial agent | <1 | <1 |

### Example 25 - Biological test

Direct contact tests were carried out with hydrogel formulations according to Example 22 with unmodified anti-microbial agents (1.0 % w/w). The Log₁₀ reduction of *P. aeruginosa* was then assessed after 0 and 5 washes with 1 mL water.

| **Anti-microbial agent** | **0 washes** | **5 washes** |
|---|---|---|
| Benzalkonium chloride | >1 | >4 |
| Chlorohexidine | >2 | >4 |
| Didecyldimethylammonium chloride | 2 | >4 |
| Sterilium with no hydrogel | >4 | 1 |
| Hydrogel without anti-microbial agent | <1 | <1 |

### Example 26 - Biological test

Direct contact tests were carried out with hydrogel formulations according to Example 22 with didecyldimethylammonium chloride (DDAC, 0.1 % w/w) in the polymerized hydrogel and was compared against other sanitizing agents on glass surfaces. The Log₁₀ reduction of *S. aureus* was then assessed after 0 and 5 washes with 1 mL water.

| **Anti-microbial agent** | **0 washes** | **5 washes** |
|---|---|---|
| DDAC retained within hydrogel formulation | >3 | >4 |
| Sterilium | >4 | 1 |
| 70 % Ethanol | >4 | <1 |
| 60 % Isopropanol | >4 | <1 |
| 5% CWS BestForm soap | 1 | <1 |

### Example 27 - Biological test

Direct contact tests were carried out with hydrogel formulations according to Example 22 with varying modified anti-microbial-agents (1 % w/w). The Log₁₀ reductions of *S. aureus* and *P. aeruginosa* were then assessed.

| **Anti-microbial agent** | ***S. aureus*** | ***P. aeruginosa*** |
|---|---|---|
| 2-(N,N,N-Dodecylmethylbenzylammonium)ethyl methacrylate bromide | >4 | 3 |
| 12-(N,N,N-Butyldimethylammonium)dodecyl methacrylate bromide | >1 | 1 |
| 2-(N,N,N-Cetyldimethylammonium)ethoxyethyl methacrylate chloride | >1 | >1 |
| 2-(N,N,N-Cetylmethylbenzylammonium)ethyl methacrylate bromide | >2 | <1 |
| 12-(N,N,N-Benzylbutylmethylammonium)dodecyl methacrylate bromide | >4 | 1 |
| 2-(N,N,N-Dodecyldimethylammonium)ethyl methacrylate bromide | 3 | 1 |
| 2-(4-Tridecylpyridin-1-uim)ethoxyethyl methacrylate chloride | >4 | 1 |
| 2-(methacryloyloxy)-N,N-dimethyl-N-(2-(2-(4-(2,4,4-trimethylpentan-2-yl)phenoxy)ethoxy)ethyl)ethan-1-aminium chloride | >4 | 1 |
| 9-(N,N,N-Benzylbutylmethylammonium)nonyl methacrylate bromide | <1 | 1 |
| 16-(N,N,N-Benzylbutylmethylammonium)cetyl methacrylate bromide | >4 | <1 |
| 2-(4-heptadecylpyridin-1-uim)ethoxyethyl methacrylate chloride | >1 | <1 |
| Hydrogel without anti-microbial agent | <1 | <1 |

### Example 28 - Biological test

Direct contact tests were carried out with hydrogel formulations according to Example 22 with 2-(methacryloyloxy)-N,N-dimethyl-N-(2-(2-(4-(2,4,4-trimethylpentan-2-yl)phenoxy)ethoxy)ethyl)ethan-1-aminium chloride (1 % w/w) and benzethonium chloride (0.1 % w/w). The Log₁₀ reduction of *S. aureus* was then assessed after 0, 5, 10, and 20 washes with 1 mL water, as well as after 2 hours of soaking in water.

| **Anti-microbial agent** | **0 washes** | **5 washes** | **20 washes** | **2 hours soaking** |
|---|---|---|---|---|
| 2-(methacryloyloxy)-N,N-dimethyl-N-(2-(2-(4-(2,4,4-trimethylpentan-2-yl)phenoxy)ethoxy)ethyl)ethan-1-aminium chloride | >4 | >3 | 4 | >3 |
| Benzethonium chloride | 2 | 3 | 2 | 3 |
| Hydrogel without anti-microbial agent | <1 | <1 | <1 | <1 |

## Claims

1. A polymerizable sanitizing formulation for forming a polymerized anti-microbial agent retaining material on a contact surface, the polymerizable sanitizing formulation comprising:
a) a polymerizable water-soluble material;
b) at least one initiator for radical polymerization of the polymerizable material to form a polymerized anti-microbial agent retaining material;
c) at least one anti-microbial agent for retention within the polymerized anti-microbial agent retaining material; and
d) at least one solvent.

2. A polymerizable sanitizing formulation as claimed in claim 1, in which the formulation is a photopolymerizable sanitizing formulation, and in which at least one initiator is at least one photoinitiator.

3. A polymerizable sanitizing formulation as claimed in either of claims 1 and 2, comprising:
a) 3 - 50 % in weight of a polymerizable water-soluble material comprising one or more materials selected from:
PEG and/or PPG and/or copolymers thereof, comprising one or more radically polymerizable end groups;
one or more natural polymeric material(s) and/or polymers derived from the Extra Cellular Matrix (ECM), such as for example one or more of: gelatin, elastin, collagen, agar/agarose, chitosan, fibrin, proteoglycans;
one or more polyamino-acid and/or derivatives thereof, preferably polylysin or gelatin methyl cellulose, carbomethyl cellulose;
one or more polysaccharides and their derivatives, preferably glycosaminoglycanes such as hyaluronic acid, chondroitinsulfate, dermatansulfate, heparansulfate, heparine, keratansulfate or alginate;
one or more nucleotides, polylipids, fatty acids, poly lactic acid, lactic acid, cationic polyallylammonium chloride; and/or
any derivative and/or fragment and/or combination thereof.
b) 0.001 - 5 % in weight of at least one initiator for radical polymerization of the polymerizable material to form a polymerized anti-microbial agent retaining material;
c) 0.001 - 10 % in weight of at least one anti-microbial agent for retention within the polymerized anti-microbial agent retaining material; and
d) 10 - 90 % in weight of at least one solvent.

4. A polymerizable sanitizing formulation as claimed in any one of claims 1 to 3, in which the polymerizable water-soluble material comprises one or more materials selected from: PEG, PPG, or copolymer moieties thereof, comprising one or more radically polymerizable end groups.

5. A polymerizable sanitizing formulation as claimed in claim 4, in which the polymerizable water-soluble material comprising PEG, PPG, or copolymer moieties thereof, comprising one or more radically polymerizable end groups, comprises one or more radically polymerizable end groups consisting of: acrylate; methacrylate; acrylamide; methacrylamide; but-3-en-2-one; inverse methacrylate of formula (I) 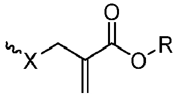 where R is an alkyl, X = O, NH; vinylsulfone; vinylurea; vinylcarbonate; vinylcarbamate; vinylthioester; vinylthiourea.

6. A polymerizable sanitizing formulation as claimed in any preceding claim, in which the initiator is a photoinitiator selected from one or more of: common UV, violet, blue or other visible light active photoinitiators or dyes.

7. A polymerizable sanitizing formulation as claimed in any preceding claim, in which the anti-microbial agent(s) comprises an amphiphilic ionic anti-microbial agent.

8. A polymerizable sanitizing formulation as claimed in claim 7, in which the or each anti-microbial agent(s) is selected from one or more of: povidone Iodine, 2-phenylphenol, chlorocresol, octenidine dihydrochloride, mecetronium ethylsulfate, didecyldimethylammonium chloride, bisbiguanide derivatives, polyhexamethylene guanidine derivatives, benzethonium chloride, quaternary ammonium compounds (QACs), for example benzalkonium chloride (Formula IV): 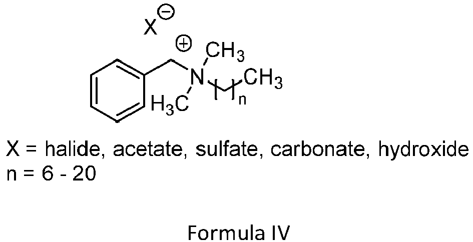 , or any combination thereof.

9. A polymerizable sanitizing formulation as claimed in any of claims 1 to 8, in which the or each anti-microbial agent has the Formula (V):
A-B-C (V)
wherein:
A is the polymerizable group selected from the group consisting of: acrylate; methacrylate; acrylamide; methacrylamide; but-3-en-2-one; inverse methacrylate of formula 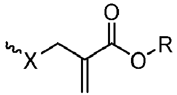 where R is an alkyl, X = O, NH; vinylsulfone; vinylurea; vinylcarbonate; vinylcarbamate; vinylthioester; vinylthiourea;
B is a linker selected among: C₂ to C₅₀ linear or branched alkyl chains, oligo(ethylene glycol): 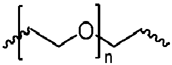 with n comprising between 1 to 3; and
C is a nitrogen-containing salt selected from the group consisting of:
i) a group having the formula 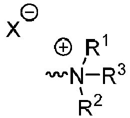
where R¹, R² and R³ can be equal or different, and are each selected from the group comprising:
benzyl, 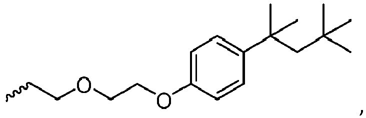 C₁ to C₅₀ linear or branched chains; and
X is selected from the group comprising: halide, sulfate, carbonate or hydroxide;
or
or ii) a group having the formula: 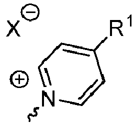
where R¹ is selected among C₁ to C₅₀ linear or branched chains; and
X is selected from the group comprising: halide, sulfate, carbonate or hydroxide.

10. Use of a polymerizable sanitisation formulation as claimed in any one of claims 1 to 9, for the formation of a polymerized anti-microbial agent retaining material on a contact surface.

11. Use of a polymerizable sanitisation formulation as claimed in any one of claims 1 to 9, for sanitisation of a contact surface.

12. Use of a polymerizable sanitisation formulation as claimed in any one of claims 1 to 9, for sanitisation of a contact surface against bilipid-containing microbe contamination.

13. Use of a polymerizable sanitisation formulation as claimed in any one of claims 1 to 9, for preventing an infection caused by microbial contamination of a contact surface.

14. A polymerized anti-microbial agent retaining material formed by polymerization of a polymerizable sanitizing formulation as claimed in any one of claims 1 to 9.

15. A method for producing a polymerizable sanitizing formulation as claimed in any one of claims 1 to 9 for forming a polymerized anti-microbial agent retaining material on a contact surface, the method comprising:
obtaining a polymerizable water-soluble material comprising PEG, PPG, or copolymer moieties thereof, comprising one or more radically polymerizable end groups;
optionally obtaining a non-polymerizable water-soluble material comprising PEG, PPG, or copolymer moieties thereof;
obtaining at least one initiator for radical polymerization of the polymerizable material to form a polymerized anti-microbial agent retaining material;
obtaining at least one anti-microbial agent for retention within the polymerized anti-microbial agent retaining material;
optionally obtaining at least one coinitiator to assist the photoinitiator in radical polymerization of the polymerizable material;
optionally obtaining at least one antioxidant; and
obtaining at least one solvent.
mixing the polymerizable water soluble material, optionally the non-polymerizable water soluble material, at least one anti-microbial agent, optionally at least one coinitiator, optionally at least one antioxidant, and at least one solvent together, and adding at least one initiator to provide a polymerizable sanitizing formulation.
